# EUROPEAN PATENT SPECIFICATION

(11) **EP 2 206 790 B1**
(45) Date of publication and mention of the grant of the patent: **26.11.2014**
(21) Application number: 10150508.9
(22) Date of filing: 12.01.2010
(51) Int. Cl.: C12Q 1/68

(54) **Method for reducing dispersion in nucleic acid amplification reaction**
Verfahren zur Verringerung der Verteilung in einer Nukleinsäurenamplifikationsreaktion
Procédé pour la réduction de la dispersion dans la réaction d'amplification d'acide nucléique

(30) Priority: 13.01.2009 JP 2009004431
(43) Date of publication of application: 14.07.2010
(73) Proprietor: FUJIFILM Corporation, Tokyo 106-8620 (JP)
(72) Inventor: Miyoshi, Hayato, Kanagawa 258-8577 (JP); Iwaki, Yoshihide, Kanagawa 258-8577 (JP)
(74) Representative: Hoffmann Eitle

(56) References cited:
- EP-A2- 0 632 134
- EP-A2- 2 058 406
- WO-A1-2006/030455
- US-A1- 2008 096 257
- NOTOMI T ET AL: "Loop-mediated isothermal amplification of DNA" NUCLEIC ACIDS RESEARCH, OXFORD UNIVERSITY PRESS, SURREY, GB, vol. 28, no. 12, 1 December 2000 (2000-12-01), pages I-VII, XP007905272 ISSN: 0305-1048
- SHERIDAN G E C ET AL: "Detection of mRNA by reverse transcription-PCR as an indicator of viability in Escherichia coli cells", APPLIED AND ENVIRONMENTAL MICROBIOLOGY, AMERICAN SOCIETY FOR MICROBIOLOGY, US, vol. 64, no. 4, 1 April 1998 (1998-04-01), pages 1313-1318, XP002194990, ISSN: 0099-2240

### SEQUENCE LISTING

<110> FUJIFILM Corporation
<120> method for reducing dispersion in nucleic acid amplification reaction
<130> FA9175A, 139 481
<150> JP 2009-004431
   <151> 2009-01-13
<160> 4
<170> PatentIn version 3.4
<210> 1
   <211> 14
   <212> DNA
   <213> Artificial
<220>
   <223> Synthetic DNA, Primer (1)
<400> 1
   ggtccagaga tacc 14
<210> 2
   <211> 16
   <212> DNA
   <213> Artificial
<220>
   <223> Synthetic DNA, Primer (2)
<400> 2
   caggctggtg gggaga 16
<210> 3
   <211> 60
   <212> DNA
   <213> Artificial
<220>
   <223> CYP2C9 gene fragment
<400> 3
   tgctgtggtg cacgaggtcc agagatacct tgaccttctc cccaccagcc tgccccatgc 60
<210> 4
   <211> 60
   <212> DNA
   <213> Artificial
<220>
   <223> CYP2C9 gene fragment
<400> 4
   gcatggggca ggctggtggg gagaaggtca acgtatctct ggacctcgtg caccacagca 60

## Description

### Technical Field

The present invention relates to a method for amplifying a nucleic acid as defined in the claims.

### Background Art

In molecular biological study, the amplification of a nucleic acid has been generally carried out by an emzymatic method using DNA polymerase. As a method for amplifying a nucleic acid, a polymerase chain reaction (PCR) has been widely known. In order to amplify a target nucleic acid sequence of interest, the PCR method comprises the following three steps: a step of denaturing double-stranded DNA used as a template to convert it to single-stranded DNA (denaturation step); a step of annealing a primer to the single-stranded DNA (annealing step); and a step of elongating a complementary strand using the primer as an origin (elongation step). In an ordinary PCR method, a thermal cycler is used, and the denaturation step, the annealing step and the elongation step are carried out at different temperatures. However, such nucleic acid amplification reaction carried out at the 3 different types of temperatures has required complicated temperature control. Moreover, the PCR method has also been problematic in that time loss increases as the number of cycles increases.

Under the aforementioned circumstances, a nucleic acid amplification method that can be carried out under isothermal conditions has been developed. Examples of such a nucleic acid amplification method include RCA (Rolling Circle Amplification: Proc. Natl. Acad. Sci, Vol. 92, 4641-4645 (1995)), ICAN (Isothermal and Chimeric primer-initiated Amplification of Nucleic acids), LAMP (Loop-Mediated Isothermal Amplification of DNA; Bio Industry, Vol. 18, No. 2 (2001)), NASBA (Nucleic acid Sequence-based Amplification method; Nature, 350, 91- (1991)), and TMA (Transcription mediated amplification method; J. Clin Microbiol. Vol. 31, 3270- (1993)).

The SDA method (JP Patent Publication (Kokai) No. 5-130870 A (1993)) is a cycling assay method using exonuclease, which is one type of amplification method for amplifying a site of interest of a target nucleic acid fragment, utilizing a polymerase elongation reaction. This is a method, which performs a polymerase elongation reaction using, as an origin, a primer specifically hybridizing with such site of interest of a target nucleic acid fragment, and at the same time, allows 5'→3' exonuclease to act thereon, so as to decompose the primer from a reverse direction. Instead of a decomposed primer, a new primer hybridizes with the site, and an elongation reaction with DNA polymerase proceeds again. This elongation reaction with polymerase and the subsequent decomposition reaction with exonuclease for dissociating the elongated strand are successively and periodically repeated. Herein, the elongation reaction with polymerase and the decomposition reaction with exonuclease can be carried out under isothermal conditions. However, in this method, exonuclease as well as polymerase should have been used. Thus, it has required high costs, and further, it has been necessary to device means of designing primers.

The LAMP method is a method for amplifying a site of interest of a target nucleic acid fragment, which has been recently developed (e.g. Notomi et al., 2000). This method uses at least 4 types of primers that complementarily recognize at least 6 specific sites of a target nucleic acid fragment and a strand-displacement-type Bst DNA polymerase that does not have 5'-3' exonuclease activity and catalyzes an elongation reaction while releasing a double-stranded DNA on a template as a single-stranded DNA, so that a site of interest of a target nucleic acid fragment can be amplified as a special structure under isothermal conditions. However, at least 4 types of primers that recognize 6 specific sites should have been used, and thus it has been extremely difficult to design such primers.

The ICAN method is also a method for amplifying a site of interest of a target nucleic acid fragment, which has been recently developed. This is a method for amplifying a gene under isothermal conditions, using an RNA-DNA chimeric primer, DNA polymerase having strand displacement activity and template exchange activity, and RNaseH. After such chimeric primer has bound to a template, a complementary strand is synthesized by DNA polymerase. Thereafter, RNaseH cleaves a chimeric primer-derived RNA portion, and from the cleavage site, an elongation reaction attended with a strand displacement reaction and a template exchange reaction takes place. By repeating this reaction, a gene is amplified. However, this method has also required the use of a special primer that is a chimeric primer, and thus it is extremely difficult to design such primer.

JP Patent Publication (Kohyo) No. 11-509406 A (1999) describes a method for amplifying DNA in a region of interest by reacting it with at least a pair of oligonucleotide primers in the presence of DNA polymerase having strand displacement ability under isothermal conditions. However, the method described in JP Patent Publication (Kohyo) No. 11-509406 A (1999) has been problematic in that it has required a comparatively long reaction time.

JP Patent Publication (Kokai) No. 2002-233379 describes a method for amplifying DNA in a region of interest by reacting it with at least a pair of oligonucleotide primers in the presence of DNA polymerase having strand displacement ability under isothermal conditions. However, the method described in JP Patent Publication (Kokai) No. 2002-233379 has been problematic in terms of a significant level of generation of non-specific amplification products.

### Summary of the Invention

It is an object of the present invention to provide a method for amplifying a nucleic acid, which does not require complicated temperature control and which can be carried out without using special enzyme or special primers. It is another object of the present invention to provide a method for amplifying a nucleic acid, in which a dispersion in the reaction rate is suppressed.

As a result of intensive studies directed towards achieving the aforementioned objects, the present inventors have succeeded in specifically amplifying a target nucleic acid sequence and further in suppressing a dispersion in the amplification rate by incubating a reaction solution at temperature (T1) necessary for high specificity in the amplification reaction, and then incubating the reaction solution at temperature (T2) necessary for high efficiency in the amplification reaction, thereby completing the present invention.

The present invention provides a method for amplifying a nucleic acid, which comprises the following steps (1) and (2):
(1) a step of incubating a reaction solution containing at least one type of deoxynucleotide triphosphate, at least one type of DNA polymerase having strand displacement activity, at least two types of oligonucleotide primers, and a nucleic acid fragment acting as a template, at temperature (T1); and
(2) a step of incubating the reaction solution at temperature (T2) that is higher than the temperature (T1) and is between 50°C and 100°C, following the step (1), and the temperature (T1) is between 10°C and 50°C or, and the reaction solution further comprises at least 0.01% or more surfactant.

Preferably, a temperature difference between the temperature (T1) and the temperature (T2) is 5°C or higher.

Preferably, the step (1) is carried out within 60 minutes.

Preferably, the step (2) is carried out within 60 minutes.

Preferably, only the 3'-terminal region of the oligonucleotide primer is substantially complementary to the nucleic acid fragment acting as a template.

Preferably, the oligonucleotide primer is substantially complementary to the nucleic acid fragment acting as a template only at one continuous site.

Preferably, the surfactant is a nonionic surfactant.

Preferably, the nonionic surfactant is selected from among polyoxyethylene sorbitan fatty acid esters and polyoxyethylene alkyl ethers.

Preferably, the reaction solution further comprises a divalent cation.

Preferably, the reaction solution further comprises a melting temperature adjuster.

Preferably, the melting temperature adjuster is dimethyl sulfoxide, betaine, formamide, or glycerol, or a mixture of two or more types thereof.

Preferably, the polymerase having a strand displacement activity is selected from the group consisting of 5'→3' exonuclease-deficient Bst. DNA polymerase derived from *Bacillus stearothermophilus,* 5'→3' exonuclease-deficient Bca DNA polymerase derived from *Bacillus caldotenax*, 5'→3' exonuclease-deficient Vent. DNA polymerase derived from *Termococcus litoralis*, and DNA polymerase derived from *Alicyclobacillus acidocaldarius.*

According to the present invention, specificity in the amplification of a target nucleic acid sequence can be increased by incubating the reaction solution at a temperature necessary for high specificity in the amplification reaction (T1), and then incubating the reaction solution at a temperature necessary for high efficiency in the amplification reaction (T2). Moreover, as a result of the increased specificity in the reaction, a dispersion in the time required for the detection of an amplification product can be reduced. Furthermore, according to the present invention, since a target nucleic acid sequence can be amplified without requiring complicated temperature control, the use of special enzyme, and complicated primer design, a method for simply and rapidly amplifying a nucleic acid with high sensitivity can be provided.

### Brief Description of the Drawings

Figure 1 shows the positional relationship of the primers used in the example with a CYP2C9 gene.
Figure 2 shows the results obtained by reacting a reaction solution A (without the incubation of step (1)) at 60°C for 60 minutes and then performing fluorescence detection over time.
Figure 3 shows the results obtained by reacting a reaction solution B (with the incubation of step (1) (at T1 = 25°C) for 10 minutes) at 60°C (= T2) for 60 minutes and then performing fluorescence detection over time.
Figure 4 shows the results obtained by reacting a reaction solution C (with the incubation of step (1) (at T1 = 25°C) for 20 minutes) at 60°C (= T2) for 60 minutes and then performing fluorescence detection over time.
Figure 5 shows the results obtained by reacting a reaction solution D (with the incubation of step (1) (at T1 = 25°C) for 30 minutes) at 60°C (= T2) for 60 minutes and then performing fluorescence detection over time.
Figure 6 shows the results obtained by performing electrophoresis by using a sample obtained by reacting the reaction solution A (without the incubation of step (1)) at 60°C (= T2) for 60 minutes.
Figure 7 shows the results obtained by performing electrophoresis by using a sample obtained by reacting the reaction solution B (with the incubation of step (1) (at T1 = 25°C) for 10 minutes) at 60°C (= T2) for 60 minutes.
Figure 8 shows the results obtained by performing electrophoresis by using a sample obtained by reacting the reaction solution C (with the incubation of step (1) (at T1 = 25°C) for 20 minutes) at 60°C (= T2) for 60 minutes.
Figure 9 shows the results obtained by performing electrophoresis by using a sample obtained by reacting the reaction solution D (with the incubation of step (1) (at T1 = 25°C) for 30 minutes) at 60°C (= T2) for 60 minutes.

### Mode for Carrying Out the Invention

Hereinafter, the present invention will be described more in detail.

The method for amplifying a nucleic acid according to the present invention comprises the following steps (1) and (2):
(1) a step of incubating a reaction solution containing at least one type of deoxynucleotide triphosphate, at least one type of DNA polymerase having strand displacement activity, at least two types of oligonucleotide primers, and a nucleic acid fragment acting as a template, at temperature (T1); and
(2) a step of incubating the reaction solution at temperature (T2) that is higher than the temperature (T1) and is between 50°C and 100°C, following the step (1).

In the present invention, amplification reaction efficiency is low at the temperature T1, and it is high at the temperature T2. At the same time, it is important that specificity in the hybridization of an oligonucleotide primer to a nucleic acid fragment acting as a template is high at the temperature T1. Under such conditions, the relationship between the temperature T1 and the temperature T2 is T1 < T2. Preferably, the difference between the temperature T1 and the temperature T2 is 5°C or more. More preferably, the difference between the temperature T1 and the temperature T2 is 10°C or more. Further preferably, the difference between the temperature T1 and the temperature T2 is 20°C or more.

The temperature T2 is between 50°C and 100°C.

In addition, the temperature T1 is between 10°C and 50°C.

Preferably, the step (1) is carried out within 60 minutes. The step (1) is carried out more preferably within 5 to 60 minutes, further preferably within 10 to 60 minutes, and still further preferably within 15 to 60 minutes.

Preferably, the step (2) is carried out within 60 minutes. The step (2) is carried out more preferably within 5 to 60 minutes, further preferably within 10 to 60 minutes, and still further preferably within 15 to 60 minutes.

The amplification method of the present invention may be carried out by any mechanism, as long as it proceeds both at the temperature (T1) and at the temperature (T2). For example, it may be possible to amplify only a region sandwiched between primers. In addition, it may also be possible to amplify a high molecular weight product by a recombination phenomenon via a nucleic acid sequence elongated with primers. Moreover, it may further be possible to amplify a high molecular weight product by a recombination phenomenon via a Tag sequence added to such primer.

Hereafter, the ingredients used in the present invention will be described below.

### (1) Deoxynucleotide triphosphate

Deoxynucleotide triphosphate is used as a substrate for an elongation reaction. Specifically, a mixture of dATP, dCTP, dGTP and dTTP is preferably used. Deoxynucleotide triphosphate may comprise analogs of dNTP (for example, 7-deaza-dGTP, etc.).

In addition, the final concentration of such deoxynucleotide triphosphate (a mixture of dATP, dCTP, dGTP and dTTP) is within the range from 0.1 mM to 3.0 mM, preferably from 0.75 mM to 3.0 mM, more preferably from 1.0 mM to 2.0 mM, and particularly preferably from 1.0 mM to 1.5 mM.

### (2) DNA polymerase

In the present invention, polymerase having strand displacement ability is used. The term "strand displacement ability" is used in the present specification to mean an activity of performing DNA replication using a nucleic acid sequence as a template while displacing a DNA strand, so as to release a complementary strand annealed to a template strand; namely an activity of performing strand displacement. Specific examples of such polymerase having strand displacement ability include 5'→3' exonuclease-deficient Bst. DNA polymerase derived from *Bacillus stearothermophilus,* 5'→3' exonuclease-deficient Bca DNA polymerase derived from *Bacillus caldotenax*, 5'→3' exonuclease-deficient Vent. DNA polymerase derived from *Termococcus litoralis*, and DNA polymerase derived from *Alicyclobacillus acidocaldarius*, but examples are not limited thereto. Such polymerase having strand displacement ability may be either a naturally-occurring protein or a recombinant protein produced by genetic engineering.

### (3) Divalent cation

In the present invention, a divalent cation is used for the metallic requirement of enzyme used, and the like. As such a divalent cation, magnesium salts, calcium salts, and other metal salts can be used. For example, magnesium chloride, magnesium acetate, magnesium sulfate and the like can be used. The final concentration of such a divalent cation is within the range preferably from 1 mM to 20 mM, and more preferably from 2 mM to 10 mM.

### (4) Surfactant

In the present invention, a surfactant is added into a reaction solution. Using such a surfactant, the advantageous effect of the present invention that is prevention of non-specific amplification of nucleic acids can be achieved. The type of the surfactant used in the present invention is not particularly limited. Examples of the surfactant that can be used in the present invention include: anionic surfactants such as sodium alkylbenzene sulfonate, sodium dodecyl sulfate (SDS), octyl sulfosuccinate or stearic acid soap; nonionic surfactants such as sucrose fatty acid ester, POE sorbitan fatty acid ester (Tween 20, Tween 40, Tween 60, Tween 80, etc.), fatty acid alkanolamide, POE alkyl ether (Brij 35, Brij 58, etc.), POE alkyl phenyl ether (Triton X-100, Triton X-114, Nonidet P40, etc.), nonylphenol, lauryl alcohol, polyethylene glycol, a polyoxyethylene-polyoxypropylene block polymer, POE alkylamine or POE fatty acid bisphenyl ether; and cationic surfactants such as cetyl pyridinium chloride, lauryl dimethyl benzyl ammonium chloride or stearyl trimethyl ammonium chloride. The amount of the surfactant used is not particularly limited, as long as the effect of the present invention can be achieved. The amount of the surfactant used is 0.01% or more, more preferably 0.05% or more, and further preferably 0.1% or more. The upper limit of the amount of the surfactant used is not particularly limited. It is generally 10% or less, preferably 5% or less, and more preferably 1% or less.

Among such surfactants, the use of nonionic surfactants is particularly preferable. Among the nonionic surfactants, a nonionic surfactant with strong hydrophilicity is preferable, and in terms of HLB value, a nonionic surfactant having an HLB value of 12 or greater is preferable. Such an HLB value is more preferably 14 or greater. The upper limit of the HLB value that is preferably applied is 20. The upper limit of the HLB value is more preferably 17 or less, and further preferably from 14 to 17. Structurally, the surfactant used in the present invention is preferably selected from among polyoxyethylene sorbitan fatty acid esters and polyoxyethylene alkyl ethers. Among such polyoxyethylene sorbitan fatty acid esters, those having only one fatty acid ester are preferable. An example of such compound is represented by the following structural formula:

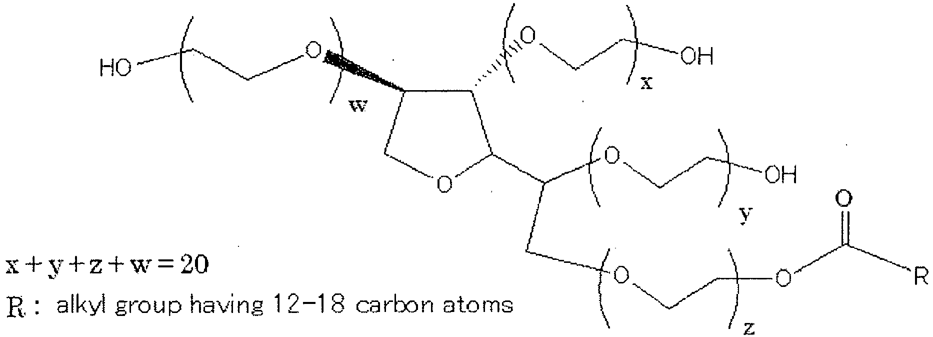

The position of an alkyl group is not particularly limited. The following structures may also be preferably used. 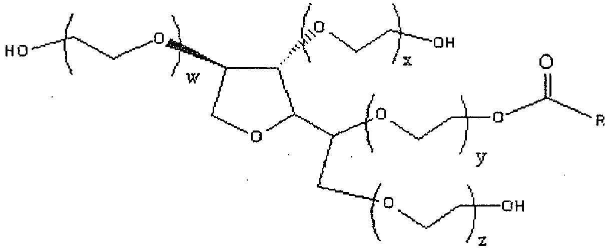 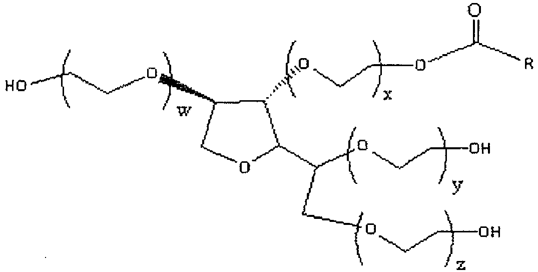 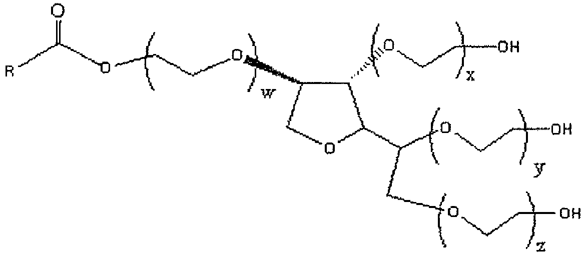 (x+y+z+w=20, R: alkyl group having 12-18 carbon atoms)

The surfactants represented by the above-descried formulae include nonionic surfactants called polyoxyethylene sorbitan fatty acid esters. Examples of such polyoxyethylene sorbitan fatty acid ester nonionic surfactants include polyoxyethylene (20) sorbitan monolaurate, polyoxyethylene (20) sorbitan monopalmitate, polyoxyethylene (20) sorbitan monostearate, and polyoxyethylene (20) sorbitan monooleate. (Product names: Tween 20, Tween 40, Tween 60, Tween 80, etc.). The amount of such a nonionic surfactant used is not particularly limited. It is preferably 0.01% or more, more preferably 0.05% or more, and further preferably 0.1% or more.

### (5) Oligonucleotide primer

The oligonucleotide used in the present invention has a nucleotide sequence substantially complementary to template DNA, and enables the elongation of a DNA strand from the 3'-terminus thereof. Thus, since the oligonucleotide has a nucleotide sequence substantially complementary to template DNA, it can be annealed to DNA used as a template. As the oligonucleotide used in the present invention, an oligonucleotide constituted with deoxyribonucleotide or ribonucleotide may be used, and such oligonucleotide may also comprise a modified ribonucleotide or a modified deoxyribonucleotide.

Preferably, only the 3'-terminal region of the oligonucleotide primer is substantially complementary to the nucleic acid fragment acting as a template.

Preferably, the oligonucleotide primer is substantially complementary to the nucleic acid fragment acting as a template only at one continuous site.

Preferably, a nucleotide sequence existing on the 3'-terminal side of a sequence substantially homologous to the 3'-terminal side portion of the oligonucleotide primer (which is a portion in which the oligonucleotide is annealed to the template nucleic acid), on the nucleotide sequence of the nucleic acid fragment acting as a template, is added as a tag to the 5'-temrinal side of the oligonucleotide primer.

The length of an oligonucleotide is not particularly limited. The length consists of generally approximately 10 to 100 nucleotides, preferably approximately 15 to 50 nucleotides, and more preferably approximately 15 to 40 nucleotides.

Such an oligonucleotide can be synthesized by a phosphoamidite method using a commercially available DNA synthesizer (for example, Model 394 DNA synthesizer manufactured by Applied Biosystems, etc.).

The amount of such an oligonucleotide used in a reaction solution is preferably 0.1 µM or more, more preferably 1 µM or more, and particularly preferably 1.5 µM or more.

### (6) Nucleic acid fragment acting as template

The nucleic acid (DNA or RNA) acting as a template in the present invention may be any one of genomic DNA, cDNA, synthetic DNA, mRNA, and total RNA. A nucleic acid prepared from a sample possibly containing a nucleic acid acting as a template may be used, or a sample possibly containing a nucleic acid acting as a template may be directly used. The type of such sample containing a nucleic acid acting as a template is not particularly limited. Examples of such sample include: body fluids (for example, whole blood, serum, urine, cerebrospinal fluid, sperm, saliva, etc.); tissues (for example, cancer tissues, etc.); organism-derived samples such as swab and a cell culture; nucleic acid-containing samples such as virus, bacteria, mold, yeast, plants, and animals; samples in which microorganisms are probably mixed (for example, food products, etc.), and samples existing in the environment, such as soil and drainage water. When a nucleic acid is prepared from the aforementioned sample, a method for preparing such nucleic acid is not particularly limited. For examples, methods known to persons skilled in the art, such as a treatment with a surfactant, an ultrasonic treatment, and purification using glass beads, may be applied. A nucleic acid may be purified from a sample by phenol extraction, chromatography, gel electrophoresis, density gradient centrifugation, and the like.

When a nucleic acid having a sequence derived from RNA is to be amplified, the method of the present invention may be carried out, using, as a template, cDNA synthesized by a reverse transcription reaction with the aforementioned RNA as a template. A primer used in such reverse transcription reaction may be either a primer having a nucleotide sequence complementary to specific template RNA, or an oligo dT primer or a primer having a random sequence. The length of such primer used in a reverse transcription is preferably approximately 6 to 100 nucleotides, and more preferably approximately 9 to 50 nucleotides. The type of enzyme used in the reverse transcription reaction is not particularly limited, as long as it has cDNA synthetic activity when RNA is used as a template. Examples of such enzyme that can be used herein include avian myeloblastosis virus reverse transcriptase (AMV RTase), Moloney murine leukemia virus reverse transcriptase (MMLV RTase), and Rous-associated virus 2 reverse transcriptase (RAV-2-RTase). Moreover, strand-displacement-type DNA polymerase also having reverse transcription activity may also be used.

In the present invention, double-stranded DNA such as genomic DNA or a nucleic acid amplification fragment or single-stranded DNA such as cDNA prepared from RNA by a reverse transcription reaction may be used as template DNA. The aforementioned double-stranded DNA may be converted to single-stranded DNA by denaturation, and may be then used for the method of the present invention. Otherwise, the double-stranded DNA may be directly used for the method of the present invention without performing such denaturation.

### (7) Pre-treatment of nucleic acid fragment acting as template

The nucleic acid acting as a template in the present invention may be used as an amplification template, after it has been subjected to a pre-treatment.

A reagent used in such pre-treatment may comprise a surfactant, an anticoagulant drug, a protease, and a lipid-degrading enzyme, for example. The liquid may be acidic or alkaline.

Such pre-treatment process may include a step of heating a nucleic acid fragment to a high temperature (for example, 98°C) or a step of treating it with a denaturing agent. Moreover, it may further comprise a step of quenching such nucleic acid fragment to a temperature of 4°C or lower, after heating it to a high temperature.

### (8) Melting temperature adjuster

A melting temperature adjuster can be added to the reaction solution in the present invention. Specific examples of such a melting temperature adjuster include dimethyl sulfoxide (DMSO), betaine, formamide, glycerol, tetraalkylammonium salts, and a mixture of two or more types of these compounds. The amount of such a melting temperature adjuster used is not particularly limited. When the melting temperature adjuster is DMSO, formamide, or glycerol, it may be generally contained at a percentage of 10% or less in a reaction solution.

Betaine or tetraalkylammonium salts may be added in a concentration of approximately 0.2 to 3.0 M, and preferably approximately 0.5 to 1.5 M to a reaction solution.

### (9) Buffer component

The reaction solution used in the present invention may comprise a buffer component. The type of such a buffer component is not particularly limited. For example, bicine, tricine, Hepes, Tris, phosphate (sodium phosphate, potassium phosphate, etc.), and the like may be used. The final concentration of a buffer component is within the range from 5 mM to 100 mM, and particularly preferably from 10 mM to 50 mM. The pH value of the buffer component depends on the optimal pH of an enzyme used in an amplification reaction. It is generally from pH 6.0 to 9.0, and particularly preferably from pH 7.0 to 9.0.

### (10) Fluorescent dye

The reaction solution used in the present invention may comprise a fluorescent dye. The type of such fluorescent dye is not particularly limited. For example, SYBR Green I and the like may be used.

### (11) Use of the method for amplifying a nucleic acid of the present invention

The method for amplifying a nucleic acid according to the present invention can be used in detection of a nucleic acid, labeling, determination of a nucleotide sequence, detection of mutation of nucleotides (including detection of single nucleotide polymorphism and the like), and the like. Since the method for amplifying a nucleic acid of the present invention does not need to use a reaction vessel capable of temperature control, an amplification reaction can be carried out using a large amount of reaction solution.

An amplification product obtained by the method for amplifying a nucleic acid of the present invention can be detected by methods known to persons skilled in the art. For example, according to gel electrophoresis, a reaction product of a specific size can be detected by staining gel with ethidium bromide. As a detection system for detecting an amplification product, fluorescence polarization, immunoassay, fluorescence energy transfer, enzyme labeling (for example, peroxidase, alkaline phosphatase, etc.), fluorescence labeling (for example, fluorescein, rhodamine, etc.), chemiluminescence, bioluminescence, and the like can be used. It is also possible to detect an amplification product using a Taqman probe or Molecular Beacon. It is further possible to detect an amplification product using a labeled nucleotide that is labeled with biotin or the like. In this case, biotin contained in the amplification product can be detected using fluorescently labeled avidin or enzyme-labeled avidin. Moreover, using a redox intercalator known to persons skilled in the art, an amplification product may be detected with electrodes. Furthermore, an amplification product may also be detected using SPR.

By detecting magnesium pyrophosphate, nucleic acid amplification may be detected. In this case, an amplification product may be detected by other methods known to persons skilled in the art, such as detection of turbidity.

The present invention will be more specifically described in the following examples. However, these examples are not intended to limit the scope of the present invention.

### Examples

### <Example 1> Amplification reaction of nucleic acid

### (1) Preparation of nucleic acid sample solution containing target nucleic acid fragment

3.0 ng of human blood-derived DNA was heated at 98°C for 3 minutes to prepare a single strand. Thereafter, a sequence in a CYP2C9 gene was amplified under the following conditions.

### <Primers>

Primers were designed to target a CYP2C9 gene. The sequences of individual primers are shown below.

| | |
|---|---|
| Primer (1) (Forward): | |
| 5'-GGTCCAGAGATACC-3' | (SEQ ID NO: 1) |
| Primer (2) (Reverse): | |
| 5'-CAGGCTGGTGGGGAGA-3' | (SEQ ID NO: 2) |

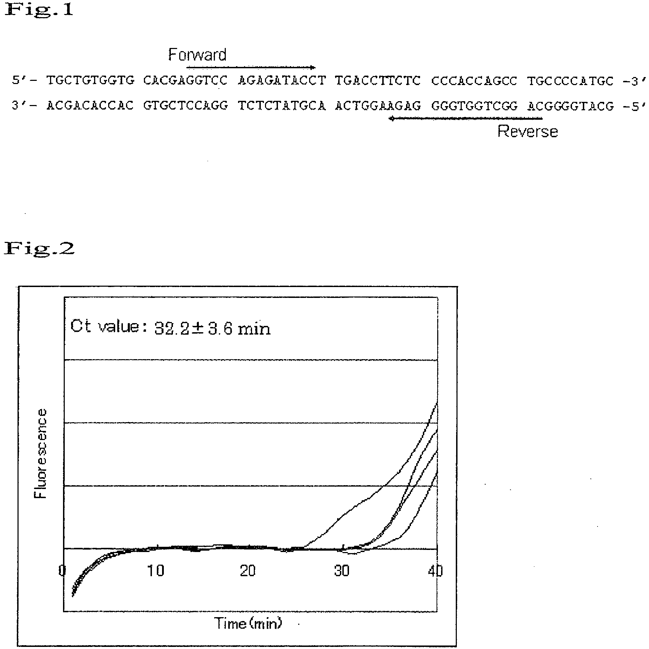

Details of the positional relationship of the aforementioned primers with the CYP2C9 gene are shown in Figure 1.

### (2) Preparation of reaction solution

A reaction solution having the following composition was prepared.

### <Composition of reaction solution>

| | |
|---|---|
| 10 × Bst Buffer (DF) | 1.0 µL |
| 100 mM MgSO4 | 0.6 µL |
| 10% (v/v) Tween 20 | 0.1 µL |
| 100% DMSO | 0.5 µL |
| 25 mM dNTP each | 0.56 µL |
| SYBR Green I (2000-times diluted) | 0.2 µL |
| 50 µM primer (1) | 0.64 µL |
| 50 µM primer (2) | 0.64 µL |
| Bst. Polymerase | 0.4 µL |
| A nucleic acid fragment sample obtained in (1) | 3.0 ng |
| Purified water | 4.96 µL |
| | 10.0 µL |

### (3) Incubation of reaction solution (step (1))

The reaction solution prepared in (2) above was incubated at room temperature (T1 = 25°C) for 0 to 30 minutes.
Reaction solution A: No incubations (0 minute)
Reaction solution B: Incubation for 10 minutes
Reaction solution C: Incubation for 20 minutes
Reaction solution D: Incubation for 30 minutes

### (4) Amplification reaction of nucleic acid (step (2))

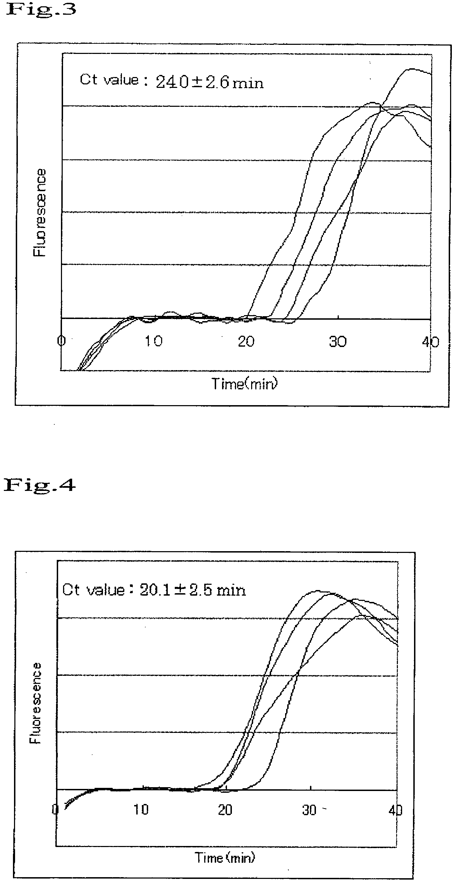

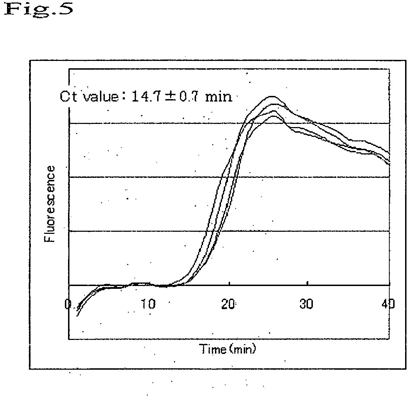

Using a real-time fluorescence detector (Mx3000p; manufactured by Stratagene), the reaction solutions obtained in (3) above were reacted at 60°C (= T2) for 60 minutes, and fluorescence was detected over time. The results regarding the reaction solution A are shown in Figure 2. The results regarding the reaction solution B are shown in Figure 3. The results regarding the reaction solution C are shown in Figure 4. The results regarding the reaction solution D are shown in Figure 5. It is to be noted that the experiment was carried out with N = 4 (that is, the experiment was carried out on four identical samples).

It is found that a nucleic acid amplification reaction from a nucleic acid sample-derived specimen can be detected in real time. Herein, the time (Ct value) at which the fluorescence level reached 250 in the aforementioned graph was calculated using Mx3000p analysis software. As a result, the results shown in Table 1 were obtained

**Table 1: T1 = 25°C, T2 = 60°C**

| Incubation time (min) | Ct value | | | | Mean Ct value | Ct value standard deviation |
|---|---|---|---|---|---|---|
| 0 | 33.1 | 33.4 | 26.9 | 35.3 | 32.2 | 3.6 |
| 10 | 25.2 | 20.8 | 26.7 | 23.5 | 24.0 | 2.6 |
| 20 | 23.6 | 19.8 | 17.6 | 19.6 | 20.1 | 2.5 |
| 30 | 13.8 | 15.2 | 14.9 | 15.1 | 14.7 | 0.7 |

A dispersion in the Ct values was reduced by establishing incubation time. That is to say, a dispersion in the amplification rate was reduced.

### (5) Electrophoresis of amplification product

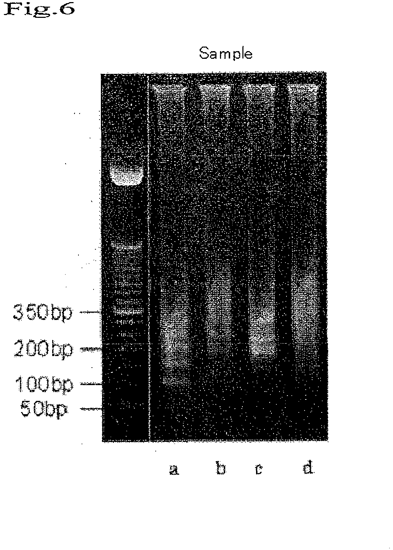

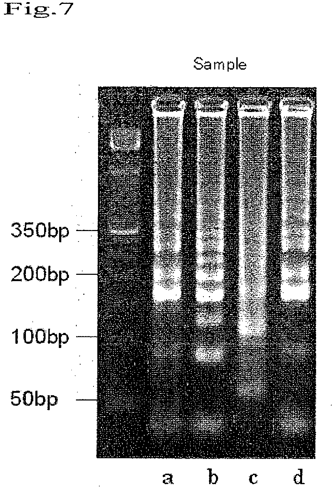

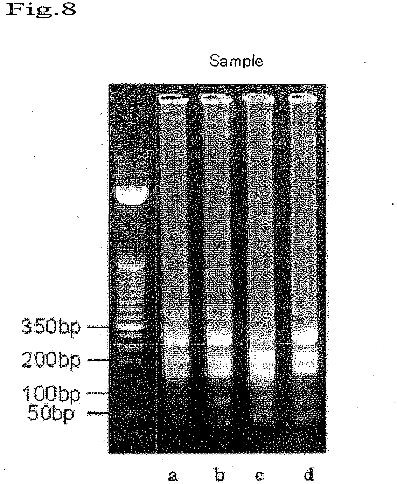

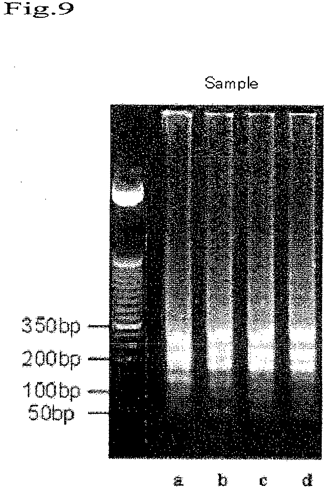

A sample obtained after the amplification reaction was electrophoresed at 100 V for 60 minutes, using 3 wt% agarose gel and a 0.5 × TBE buffer (50 mM Tris, 45 mM Boric acid, 0.5 mM EDTA, pH8.4). The results regarding the reaction solution A are shown in Figure 6. The results regarding the reaction solution B are shown in Figure 7. The results regarding the reaction solution C are shown in Figure 8. The results regarding the reaction solution D are shown in Figure 9.

In the case of samples that had not been incubated (Figure 6), they exhibited different band patterns (N = 4). On the other hand, in the case of samples that had been incubated for 10 minutes (Figure 7), two out of the four samples (samples a and d) exhibited the same band patterns. Moreover, in the case of samples that had been incubated for 20 minutes (Figure 8) and for 30 minutes (Figure 9), all the samples exhibited almost the same patterns (N = 4).

From the results of electrophoretic patterns, it was found that amplification products generated as a result of incubation also became uniform. That is, it was found that specificity in the amplification reaction was enhanced.

### <Example 2> Change in incubation temperature

### (1) Preparation of nucleic acid sample solution containing target nucleic acid fragment

3.0 ng of human blood-derived DNA was heated at 98°C for 3 minutes to prepare a single strand. Thereafter, a sequence in a CYP2C9 gene was amplified under the following conditions.

### <Primers>

Primers were designed to target a CYP2C9 gene. The sequences of individual primers are shown below.

| | |
|---|---|
| Primer (1) (Forward): | |
| 5'-GGTCCAGAGATACC-3' | (SEQ ID NO:1) |
| Primer (2) (Reverse): | |
| 5'-CAGGCTGGTGGGGAGA-3' | (SEQ ID NO: 2) |

Details of the positional relationship of the aforementioned primers with the CYP2C9 gene are shown in Figure 1.

### (2) Preparation of reaction solution

A reaction solution having the following composition was prepared.

### <Composition of reaction solution>

| | |
|---|---|
| 10 × Bst Buffer (DF) | 1.0 µL |
| 100 mM MgSO4 | 0.6 µL |
| 10% (v/v) Tween 20 | 0.1 µL |
| 100% DMSO | 0.5 µL |
| 25 mM dNTP each | 0.56 µL |
| SYBR Green I (2000-times diluted) | 0.2 µL |
| 50 µM primer (1) | 0.64 µL |
| 50 µM primer (2) | 0.64 µL |
| Bst. Polymerase | 0.4 µL |
| A nucleic acid fragment sample obtained in (1) | 3.0 ng |
| Purified water | 4.96 µL |
| | 10.0 µL |

### (3) Incubation of reaction solution (step (1))

The reaction solution prepared in (2) above was incubated at (T1 =) 30°C, 40°C or 50°C, for 5,10 or 15 minutes.

### (4) Amplification reaction of nucleic acid (step (2))

Using a real-time fluorescence detector (Mx3000p; manufactured by Stratagene), the reaction solution obtained in (3) above was reacted at 60°C (= T2) for 60 minutes, and fluorescence was detected over time. The experiment was carried out with N = 4 (that is, the experiment was carried out on four identical samples).

It is found that a nucleic acid amplification reaction from a nucleic acid sample-derived specimen can be detected in real time. Herein, the time (Ct value) at which the fluorescence level reached 250 in the aforementioned graph was calculated using Mx3000p analysis software. As a result, the results shown in Tables 2 to 4 were obtained.

A dispersion in the amplification rate was evaluated with standard deviation of the Ct value. The standard deviations obtained by incubation at 30°C, 40°C and 50°C for 15 minutes or less were each smaller than the standard deviation (3.6 minutes) obtained without incubation. That is to say, a dispersion in the amplification rate was reduced by performing incubation.

**Table 2: T1 = 30°C, T2 = 60°C**

| Incubation time (min) | Ct value | | | | Mean Ct value | Ct value standard deviation |
|---|---|---|---|---|---|---|
| 5 | 25.9 | 25.6 | 26.8 | 26.9 | 26.3 | 0.7 |
| 10 | 17.5 | 19.8 | 21.1 | 24.2 | 20.7 | 2.8 |
| 15 | 20.1 | 20.9 | 21.8 | 19.6 | 20.6 | 0.9 |

**Table 3: T1 = 40°C, T2 = 60°C**

| Incubation time (min) | Ct value | | | | Mean Ct value | Ct value standard deviation |
|---|---|---|---|---|---|---|
| 5 | 15.7 | 23.1 | 20.6 | 22.7 | 20.5 | 3.4 |
| 10 | 18.4 | 18.1 | 16.9 | 17.4 | 17.7 | 0.7 |
| 15 | 16.1 | 14.2 | 15.9 | 15.9 | 15.5 | 0.9 |

**Table 4: T1 = 50°C, T2 = 60°C**

| Incubation time (min) | Ct value | | | | Mean Ct value | Ct value standard deviation |
|---|---|---|---|---|---|---|
| 5 | 23.8 | 22.9 | 20.7 | 22.5 | 22.5 | 1.3 |
| 10 | 17.8 | 17.3 | 15.8 | 12.0 | 15.7 | 2.6 |
| 15 | 8.2 | 13.9 | 9.8 | 12.8 | 11.1 | 2.6 |

## Claims

1. A method for amplifying a nucleic acid, which comprises the following steps (1) and (2):
(1) a step of incubating a reaction solution containing at least one type of deoxynucleotide triphosphate, at least one type of DNA polymerase having strand displacement activity, at least two types of oligonucleotide primers, and a nucleic acid fragment acting as a template, at temperature (T1); and
(2) a step of incubating the reaction solution at temperature (T2) that is higher than the temperature (T1) and is between 50°C to 100°C, following the step (1)
wherein the temperature (T1) is between 10°C and 50°C, and the reaction solution further comprises at least 0.01% or more surfactant,
provided that a case is excluded where
human liver total RNA is used as template RNA, a forward primer having the sequence 5'-GGGCATGGGTCAGAAGGATT-3' and a reverse primer having the sequence 5'-CCTCGTCGCCCACATAG-3' is used, the reaction solution is incubated at 25°C for 10 minutes, at 42°C for 50 minutes, and at 60°C for 60 minutes, the reaction solution having the composition:
| | |
|---|---|
| Template RNA (0.1 µg/µL) | 1.0 µL |
| Random 6mer Primer (100µM) | 1.0 µL |
| Dithiothreitol (0.1 µM) | 2.0 µL |
| 10 x Bst Buffer | 2.5 µL |
| MgSO₄ (100 mM) | 1.5 µL |
| Tween20 (10% (v/v)) | 0.25 µL |
| DMSO | 1.25 µL |
| dNTP (25 mM each) | 1.8 µL |
| SYBR Green I (2000-fold diluted) | 0.5 µL |
| 50 µM primer (1) | 1.6 µL |
| 50 µM primer (2) | 1.6 µL |
| RTase (SuperScript II) | 1.0 µL |
| Bst. Polymerase | 1.0 µL |
| Purified water | 17.0 µL |
| | 25.0 µ |

2. The method according to claim 1, wherein a temperature difference between the temperature (T1) and the temperature (T2) is 5°C or higher.

3. The method according to claim 1, wherein the step (1) is carried out within 60 minutes.

4. The method according to claim 1, wherein the step (2) is carried out within 60 minutes.

5. The method according to claim 1, wherein only the 3'-terminal region of the oligonucleotide primer is substantially complementary to the nucleic acid fragment acting as a template.

6. The method according to claim 5, wherein the oligonucleotide primer is substantially complementary to the nucleic acid fragment acting as a template only at one continuous site.

7. The method according to claim 1, wherein the surfactant is a nonionic surfactant.

8. The method according to claim 7, wherein the nonionic surfactant is selected from among polyoxyethylene sorbitan fatty acid esters and polyoxyethylene alkyl ethers.

9. The method according to claim 1, wherein the reaction solution further comprises a divalent cation.

10. The method according to claim 1, wherein the reaction solution further comprises a melting temperature adjuster.

11. The method according to claim 10, wherein the melting temperature adjuster is dimethyl sulfoxide, betaine, formamide, or glycerol, or a mixture of two or more types thereof.

12. The method according to claim 1, wherein the polymerase having a strand displacement activity is selected from the group consisting of 5'->3' exonuclease-deficient Bst. DNA polymerase derived from *Bacillus stearothermophilus,* 5'->3' exonuclease-deficient Bca DNA polymerase derived from *Bacillus caldotenax,* 5'->3' exonuclease-deficient Vent. DNA polymerase derived from *Termococcus litoralis,* and DNA polymerase derived from *Alicyclobacillus acidocaldarius.*

## Patentansprüche

1. Verfahren zur Vervielfältigung einer Nukleinsäure, welches die folgenden Schritte (1) und (2) umfasst:
(1) einen Schritt der Inkubation einer Reaktionslösung bei Temperatur (T1) wobei die Reaktionslösung mindestens eine Art von Desoxynukleotidtriphosphat, mindestens eine Art von DNS-Polymerase mit Strang verdrängender Aktivität, mindestens zwei Arten von Oligonukleotidprimern und ein Nukleinsäurefragment, welches als Vorlage dient, enthält; und
(2) einen Schritt der Inkubation der Reaktionslösung bei Temperatur (T2), die höher ist als Temperatur (T1) und zwischen 50°C bis 100°C liegt, wobei Schritt (2) auf Schritt (1) folgt,
wobei die Temperatur (T1) zwischen 10°C und 50°C liegt und die Reaktionslösung weiterhin mindestens 0,01% oder mehr Tensid umfasst,
wobei ein Fall ausgeschlossen ist, bei dem gesamte RNS menschlicher Leber als Vorlage-RNS benutzt wird, ein Vorwärtsprimer mit der Sequenz 5'-GGGCATGGGTCAGAAGGATT-3' und ein Rückwärtsprimer mit der Sequenz 5'-CCTCGTCGCCCACATAG-3' benutzt wird, die Reaktionslösung bei 25°C für 10 Minuten, bei 42°C für 50 Minuten und bei 60°C für 60 Minuten inkubiert wird, wobei die Reaktionslösung folgende Zusammensetzung hat:
| | |
|---|---|
| Vorlage-RNS (0,1 µg/µL) | 1,0 µL |
| "Random 6mer" Primer (100 µM) | 1,0 µL |
| Dithiothreitol (0,1 µM) | 2,0 µL |
| 10 × Bst Puffer | 2,5 µL |
| MgSO₄ (100 mM) | 1,5 µL |
| Tween20 (10% (v/v)) | 0,25 µL |
| DMSO | 1,25 µL |
| dNTP (jeweils 25 mM) | 1,8 µL |
| SYBR Green I (2000-fach verdünnt) | 0,5 µL |
| 50 µM Primer (1) | 1,6 µL |
| 50 µM Primer (2) | 1,6 µL |
| RTase (SuperScript II) | 1,0 µL |
| Bst. Polymerase | 1,0 µL |
| Gereinigtes Wasser | 17,0 µL |
| | 25,0 µL |

2. Verfahren gemäß Anspruch 1, wobei ein Temperaturunterschied zwischen der Temperatur (T1) und der Temperatur (T2) 5°C oder höher ist.

3. Verfahren gemäß Anspruch 1, wobei der Schritt (1) innerhalb von 60 Minuten ausgeführt wird.

4. Verfahren gemäß Anspruch 1, wobei der Schritt (2) innerhalb von 60 Minuten ausgeführt wird.

5. Verfahren gemäß Anspruch 1, wobei nur die 3'-terminale Region des Oligonukleotidprimers zu dem Nukleinsäurefragment, welches als Vorlage dient, im Wesentlichen komplementär ist.

6. Verfahren gemäß Anspruch 5, wobei der Oligonukleotidprimer nur an einer fortlaufenden Stelle zu dem Nukleinsäurefragment, welches als Vorlage dient, im Wesentlichen komplementär ist.

7. Verfahren gemäß Anspruch 1, wobei das Tensid ein nichtionisches Tensid ist.

8. Verfahren gemäß Anspruch 7, wobei das nichtionische Tensid unter Polyoxyethylensorbitan-Fettsäureestern und Polyoxyethylen-Alkylethern ausgewählt ist.

9. Verfahren gemäß Anspruch 1, wobei die Reaktionslösung weiterhin ein zweiwertiges Kation umfasst.

10. Verfahren gemäß Anspruch 1, wobei die Reaktionslösung weiterhin ein Mittel zur Anpassung der Schmelztemperatur umfasst.

11. Verfahren gemäß Anspruch 10, wobei das Mittel zur Anpassung der Schmelztemperatur Dimethylsulfoxid, Betain, Formamid oder Glyzerin oder eine Mischung aus zwei oder mehr Arten hiervon ist.

12. Verfahren gemäß Anspruch 1, wobei die Polymerase mit einer Strang verdrängenden Aktivität ausgewählt ist aus der Gruppe, bestehend aus von Bacillus stearothermophilus stammender 5'->3' Exonukleasedefizienter Bst.-DNS-Polymerase, von Bacillus caldotenax stammender 5'->3' Exonuklease-defizienter Bca-DNS-Polymerase, von Thermococcus litoralis stammender 5'->3' Exonuklease-defizienter Vent.-DNS-Polymerase und von Alicyclobacillus acidocaldarius stammender DNS-Polymerase.

## Revendications

1. Procédé pour amplifier un acide nucléique qui comprend les étapes (1) et (2) suivantes :
(1) une étape d'incubation d'une solution réactionnelle contenant au moins un type de désoxynucléotide triphosphate, au moins un type d'ADN polymérase ayant une activité de déplacement de brin, au moins deux types d'amorces oligonucléotidiques, et un fragment d'acide nucléique jouant le rôle de matrice, à une température (T1) ; et
(2) une étape d'incubation de la solution réactionnelle à une température (T2) qui est supérieure à la température (T1) et est entre 50°C et 100°C, après l'étape (1)
où la température (T1) est entre 10°C et 50°C, et la solution réactionnelle comprend en outre au moins 0,01 % ou plus de tensioactif,
à condition qu'un cas soit exclu où
l'ARN total de foie humain est utilisé comme ARN formant matrice, une amorce sens ayant la séquence 5'-GGGCATGGGTCAGAAGGATT-3' et une amorce antisens ayant la séquence 5'-CCTCGTCGCCCACATAG-3' sont utilisées, la solution réactionnelle est incubée à 25°C pendant 10 minutes, à 42°C pendant 50 minutes et à 60°C pendant 60 minutes, la solution réactionnelle ayant la composition :
| | |
|---|---|
| ARN formant matrice (0,1 µg/µL) | 1,0 µL |
| Amorce 6mère aléatoire (100 µM) | 1,0 µL |
| Dithiothréitol (0,1 µM) | 2,0 µL |
| 10 x tampon de Bst | 2,5 µL |
| MgSO₄ (100 mM) | 1,5 µL |
| Tween20 (10 % (v/v)) | 0,25 µL |
| DMSO | 1,25 µL |
| dNTP (25 mM chacun) | 1,8 µL |
| SYBR Green I (dilué 2000 fois) | 0,5 µL |
| amorce (1) 50 µM | 1,6 µL |
| amorce (2) 50 µM | 1,6 µL |
| RTase (SuperScript II) | 1,0 µL |
| Polymérase de Bst. | 1,0 µL |
| Eau purifiée | 17,0 µL |
| | 25,0 µ |

2. Procédé selon la revendication 1, où une différence de température entre la température (T1) et la température (T2) est 5°C ou plus.

3. Procédé selon la revendication 1, où l'étape (1) est accomplie en 60 minutes.

4. Procédé selon la revendication 1, où l'étape (2) est accomplie en 60 minutes.

5. Procédé selon la revendication 1, où seule la région 3'-terminale de l'amorce oligonucléotidique est sensiblement complémentaire du fragment d'acide nucléique jouant le rôle de matrice.

6. Procédé selon la revendication 5, où l'amorce oligonucléotidique est sensiblement complémentaire du fragment d'acide nucléique jouant le rôle de matrice seulement à un site continu.

7. Procédé selon la revendication 1, où le tensioactif est un tensioactif non ionique.

8. Procédé selon la revendication 7, où le tensioactif non ionique est choisi parmi les esters d'acides gras et de sorbitane polyoxyéthyléné et les alkyléthers polyoxyéthylénés.

9. Procédé selon la revendication 1, où la solution réactionnelle comprend en outre un cation divalent.

10. Procédé selon la revendication 1, où la solution réactionnelle comprend en outre un agent d'ajustement de la température de fusion.

11. Procédé selon la revendication 10, où l'agent d'ajustement de la température de fusion est le diméthylsulfoxyde, la bétaïne, le formamide ou le glycérol, ou un mélange de deux ou plusieurs types de ceux-ci.

12. Procédé selon la revendication 1, où la polymérase ayant une activité de déplacement de brin est choisie dans le groupe consistant en l'ADN polymérase de Bst. déficiente en 5'→3' exonucléase dérivée de *Bacillus stearothermophilus,* l'ADN polymérase de Bca déficiente en 5'→3' exonucléase dérivée de *Bacillus caldotenax,* l'ADN polymérase de Vent. déficiente en 5'→3' exonucléase dérivée de *Termococcus litoralis,* et l'ADN polymérase dérivée de *Alicyclobacillus acidocaldarius*
